## Europäisches Patentamt

## European Patent Office

## Office européen des brevets

(11) Publication number: **0 065 137**
**B1**

(12) **EUROPEAN PATENT SPECIFICATION**

(45) Date of publication of patent specification: **18.09.85**

(51) Int. Cl.⁴: **C 12 Q 1/04**

(21) Application number: **82103604.3**

(22) Date of filing: **28.04.82**

(54) **Culture medium composition.**

(30) Priority: **15.05.81 JP 72920/81**

(43) Date of publication of application:
**24.11.82 Bulletin 82/47**

(45) Publication of the grant of the patent:
**18.09.85 Bulletin 85/38**

(84) Designated Contracting States:
**CH DE FR GB LI NL SE**

(56) References cited:
**DE-A-2 212 573**
**DE-A-2 941 471**
**GB-A-2 059 059**
**US-A-3 902 969**

(73) Proprietor: **Showa Yakuhin Kako Co., Ltd.**
**No. 16-5, 1-chome, Kyobashi**
**Chuo-ku Tokyo (JP)**
(73) Proprietor: **Takazoe, Ichiro**
**No. 7-17-18-231, Shinjuku Shinjuku-ku**
**Tokyo (JP)**
(73) Proprietor: **Takeuchi, Mitsuharu**
**No. 2-26-19, Ichikawa**
**Ichikawa-shi Chiba-ken (JP)**
(73) Proprietor: **Ohta, Kosei**
**No. 3-13-17-205, Ogikubo Suginami-ku**
**Tokyo (JP)**
(73) Proprietor: **Matsukubo, Takashi**
**No. 2-15-34-302, Takanawa Minato-ku**
**Tokyo (JP)**

(72) Inventor: **Matsukubo, Takashi**
**No. 2-15-34-302, Takanawa Minato-ku**
**Tokyo (JP)**
Inventor: **Ohta, Kosei**
**No. 3-13-17-205, Ogikubo Suginami-ku**
**Tokyo (JP)**
Inventor: **Takeuchi, Mitsuharu**
**No. 2-26-19, Ichikawa Ichikawa-shi**
**Chiba-ken (JP)**
Inventor: **Takazoe, Ichiro**
**No. 7-17-18-231, Shinjuku Shinjuku-ku**
**Tokyo (JP)**

Courier Press, Leamington Spa, England.

(72) Inventor: Saito, Hitoshi
No. 141-4, Sasanodai Asahi-ku
Yokohama-shi Kanagawa-ken (JP)
Inventor: Tanimura, Masayuki
No. 2-14-22 Taishido Setagaya-ku
Tokyo (JP)
Inventor: Matsumoto, Kiyoyuki
No. 1589-21, Uchikoshi-machi Hachiohji-shi
Tokyo (JP)
Inventor: Asami, Kuniaki
No. 1-16-10-202, Minami-Kugahara Ohta-ku
Tokyo (JP)


(74) Representative: Hoormann, Walter, Dr. et al
FORRESTER & BOEHMERT Widenmayerstrasse
4/I
D-8000 München 22 (DE)

2

## Description

The invention is concerned with a culture medium composition for propagating and optionally testing the presence of Streptococcus mutans, comprising an aqueous solution of main nutritional ingredients of an MSB broth including tryptose(s), peptone(s), sucrose, dextrose, phosphate and additives, comprising antibiotics, potassium-tellurite and at least one coloring material.

The present invention relates to a culture medium for testing the dental caries activity, in which two or three of ingredients of a liquid medium capable of selectively propagating bacteria which causes dental caries, *Streptococcus mutans,* are contained. These ingredients are not added to said culture medium in the initial stage but are present outside in the dry state and just before use of said culture medium, said ingredients are included in said culture medium to obtain a desirable composition of the culture medium.

Background of the invention

The human tooth decay is a so-called multi-factorial disease which is caused by many factors which are connected with one another in a complicated manner. One of the main factors is that dental plaque is formed from saliva and sucrose taken by streptococci always present in the mouth and tooth decay is readily caused by this dental plaque. As the streptococci synthesizing this dental plaque, there are known *Streptococcus sanguis, Streptococcus mutans* and *Streptococcus salivarius.* Among these streptococci, it is *Streptococcus mutans* that synthesizes water-insoluble polysaccharides and forms dental plaque inducing the dental caries. Accordingly, in order to prevent the dental caries, it is important to know the environmental condition in the mouth, especially the amount of *Streptococcus mutans* present in the mouth. For this purpose, there was previously developed and proposed a method of measuring the dental caries activity (see Japanese Patent Application Laid-Open Specification No. 55260/1980). The MSB broth that is used in this conventional method, however, has several defects as described below. More specifically, it has been found that the composition of the mitis salivarius bacitracin-broth is changed with the lapse of time after preparation of the mitis salivarius bacitracin-broth and propagation of Streptococcus mutans (hereinafter referred to as "dental caries pathogen") and inhibition of growth of other bacteria become uncertain. It has been considered that this defect is based on the essence of the composition of the mitis salivarius bacitracin-broth and is therefore unavoidable. The reason is that it is impossible to store an antibiotic substance such as Bacitracin at normal temperatures in the culture medium for a long time without reduction of the antibiotic activity thereof. Accordingly, a fresh mitis salivarius bacitracin-broth is prepared when the test is carried out. Therefore, in the clinical examination, it is practically impossible for a dental practitioner to apply the above-mentioned method using this mitis salivarius bacitracin-broth to diagnosis.

The present invention relates to a culture medium composition for propagating and optionally testing the presence of Streptococcus mutans comprising an aqueous solution of main nutritional ingredients of an MSB broth including tryptose(s), peptone(s), sucrose, dextrose, phosphate and additives, comprising antibiotics, potassium-tellurite and at least one coloring material, characterized by providing the additive reagents, comprising bacitracin (antibiotic substance), potassium tellurite and at least one coloring material in dried form on (a) support(s); and providing a sterile liquid basal medium, comprising the main nutritional dissolved in water in a separate vessel.

This means, that a culture medium for selectively propagating streptococcus mutans, which causes dental caries, is provided that way that some ingredients are not added to the liquid parts culture medium in storage form, but are present outside of the liquid medium in the dry state and (just before use) said ingredients are transferred into said liquid medium to obtain the desired composition of the culture medium.

We made researches with a view to provide an improved simple mitis salivarius bacitracin-broth having the same capacity as that of the original mitis salivarius bacitracin-broth which would be conveniently used by practising dentists in an ordinary clinical examination and would be manufactured at a low cost. The composition of the mitis salivarius bacitracin-broth medium and the culture medium of the present invention are shown in Table 1.

# 0 065 137

## TABLE 1
### Compositions (g/1000 ml) of MSB broth and culture medium of present invention

| Ingredients | Mitis salivarius bacitracin-broth | Culture medium of present invention | |
|---|---|---|---|
| Tryptose[1] | 10 | 10 ⎱ | |
| Peptone[2] | 10 | 10 | |
| Glucose | 1 | 1 | liquid basal medium |
| Sucrose | 200 | 200 | |
| Dipotassium phosphate | 4 | 4 ⎰ | |
| | | | |
| Bacitracin | 200 u | 200 u ⎱ | additive reagents |
| Potassium tellurite | 0.01 | 0.01 | (kept in dry state |
| Crystal violet[3] | 0.0008 | 0.0008 | outside until just |
| Trypan blue | 0.075 | 0.075 ⎰ | before use) |

Note

[1] Bacto tryptose (Difco)

[2] Proteose peptone No. 3 (Difco)

[3] Crystal violet may be included in the liquid basal medium. We carefully examined mutual reactions among the respective ingredients of the mitis salivarius bacitracin-broth and as the result we have created a combination of culture medium composition as described below.

A liquid basal medium having the composition from which Bacitracin (hereinafter referred to as "BA"), potassium tellurite (hereinafter referred to as "TE"), and Trypan Blue (hereinafter referred to as "TB") and/or Crystal Violet, have been removed is prepared, and this liquid basal medium is sterilized and is stored aseptically. Separately, BA, TE and TB are made independently present as additive reagents in the dry state on an appropriate support, and the additive reagent-containing support is sterilized and is stored aseptically. When the test is actually carried out, a dentist dissolves the additive reagents in the liquid basal medium sterilely to form an instant mitis salivarius bacitracin-broth simply. In this case, TB, a coloring material which is contained in the support is dissolved in the liquid basal medium to color the medium. By this coloration, completion of dissolution of other ingredients can be confirmed. From the results of the experiments made by us, it was found that the above-mentioned combination of culture medium composition is comparable to the conventional mitis salivarius bacitracin-broth as a clinical medium for selectively culturing the dental caries pathogen in saliva (see Table 2) and that even if the combination of culture medium composition is subjected to 6 months' shelf life test, the capacity is not degraded (see Table 3). We have now completed the present invention based on the foregoing findings.

4

**0 065 137**

TABLE 2

Results of determination of caries activity by MSB broth
and culture medium of present invention

| Sample | Mitis salivarius bacitracin-broth | Present invention |
|---|---|---|
| 1 | + | + |
| 2 | +++ | +++ |
| 3 | − | − |
| 4 | +++ | +++ |
| 5 | +++ | +++ |
| 6 | +++ | +++ |
| 7 | + | + |
| 8 | − | − |
| 9 | ++ | ++ |
| 10 | + | + |

Note

Mark "−" indicates no propagation of the dental caries pathogen, and each of marks "+" through "+++" indicates the degree of propagation of the dental caries pathogen, which has a relation to the number of cells of the bacteria in saliva.

Test method:

0.1 ml each of saliva collected from 10 healthy adults was added to 2 ml of the ordinary mitis salivarius bacitracin-broth and to one sample of the culture medium described in Example 2 given hereinafter, respectively, and cultured at 37°C for 24 hours according to routine procedures. The degree of propagation was determined by the naked eye examination according to the standard customarily adopted in this field.

TABLE 3

Capacity of culture medium of present invention after shelf test

| Times of repetition of test | Shelf test period (months) | | | |
|---|---|---|---|---|
| | 0 (control) | 2 | 4 | 6 |
| 1 | +++ | +++ | +++ | +++ |
| 2 | +++ | +++ | +++ | +++ |
| 3 | +++ | +++ | +++ | +++ |

Test method:
Culture medium:
Trial product Lot No. 5402 prepared in Example 1 given hereinafter.

Shelf life test:
Trial product Lot No. 5402 was stored in an incubator maintained at 40°C. A part of the products was taken out at an interval of 2 months, and immediately stored in a freezer maintained below −5°C.

Control:
Trial product Lot No. 5402 was stored in a freezer maintained below −5°C directly without the shelf life test.

Sample (fresh saliva):
Saliva of a 23-years-old healthy man in whom high dental caries activity was found by a preliminary test.

5

**0 065 137**

Operation:

0.1 ml of the sample was added to the culture medium (control culture medium or culture medium subjected to the shelf life test), and cultured at 37°C for 24 hours according to routine procedures.

In carrying out the present invention, the vessel in which the liquid basal medium is contained functions also as a culturing device, and therefore, a colorless transparent glass vessel having an inner diameter of about 10 mm, for example, an ampoule, a vial, a tubing bottle or a test tube, can advantageously be used. The additive reagents, that is, BA, TE and TB, are independently freeze-dried or spread on a paper such as a filter paper. In each case, solvent is eliminated as much as possible to obtain a product of the present invention.

The present invention will now be described in detail with reference to the following Examples. Incidentally, the liquid basal medium shown in Table 1 was commonly used in these Examples.

Example 1

(A) A colorless transparent glass vial having an inner diameter of 12 mm and a diameter of about 8 mm in the vicinity of the opening was charged with 2 ml of the liquid basal medium and the glass vial was sealed with a rubber stopper. Sterilization was performed at 115°C for 15 minutes.

(B) A circular filter paper having a thickness of 0.8 mm and a diameter of 8 mm was used as the support for the additive reagents. The additive reagents, BA, TE and TB were independently dissolved and included in the support in manners as described below, and the support was dried under reduced pressure and sealed in a vessel.

1) BA:

A solution of 0.4 U of Bacitracin in about 0.002 ml of water was poured onto the support by using a micropipette.

2) TE:

A solution of 0.4 mg of potassium tellurite in about 0.002 ml of water was poured onto a different support by using a micropipette.

3) TB:

A solution of 0.15 mg of Trypan Blue in about 0.002 ml of water was poured onto a different support by using a micropipette.

When the culture medium composition was actually used, the rubber stopper was taken out from the vessel containing the basal medium, which was obtained in (A) above, and the reagent-containing supports obtained in (B-1), (B-2) and (B-3) above were thrown into the basal medium described in (A) above. After 30 seconds, the supports were taken out and 0.1 ml of saliva of the patient was added to the culture medium. Then, the vessel was sealed again with the rubber stopper and cultivation was carried out at 37°C for 24 hours.

Example 2

(A) A 3 ml-capacity ampoule having an inner diameter of about 9.5 mm and an opening diameter of about 4 mm (the diameter of the broken face on the tubular body top when the ampoule was broken) was charged with 2 ml of the liquid basal medium, and the top end of the ampoule was melt-sealed and sterilization was carried out at 115°C for 15 minutes.

(B) A square filter paper having a thickness of 0.5 mm and one side of 3.5 mm was used as the support. Solutions of the additive reagents BA, TE and TB were independently poured onto supports in the same manner as described in (B) of Example 1 to obtain a product.

When the culture medium composition was actually used, the ampoule (A) was broken, and the reagent-containing paper supports (B-1), (B-2) and (B-3) were added to the basal medium (A) in the same manner as described in Example 1. Then, 0.1 ml of saliva of the patient was added to the medium and the opening was covered with a sterilized aluminum cap. Cultivation was carried out at 37°C for 24 hours.

Example 3

(A) A basal medium was prepared in the same manner as in (A) of Example 1 except that Crystal Violet was not added. Then, 2 ml of the basal medium was charged in a small glass tube having an inner diameter of about 10 mm and a length of 60 mm, and the opening was covered with a closely fitting polypropylene stopper. Sterilization was carried out at 115°C for 15 minutes.

(B) Additive reagent-containing three square filter paper supports having a thickness of 0.5 mm and one side of 3.5 mm were prepared in the same manner as described in Example 2, and they were bonded in a line to a transparent plastic sheet having a width of 3.5 mm, a length of 50 mm and a thickness of 0.3 mm by using an appropriate adhesive. In this case, the three supports were not separated from one another but combined together, and therefore, they could be handled very easily.

The BA and TE supports were prepared in the same manner as described in Example 2, but the TB support was prepared in a manner as described in 3) below.

6

3) TB:

A mixed coloring material solution of 0.15 mg of Trypan Blue and 0.0016 mg of Crystal Violet in about 0.002 ml of water was poured onto the support (filter paper) by using a micropipette.

## Claims

1. A culture medium composition for propagating and optionally testing the presence of Streptococcus mutans, comprising an aqueous solution of main nutritional ingredients of an MSB broth including tryptose(s), peptone(s), sucrose, dextrose, phosphate and additives, comprising antibiotics, potassium-tellurite and at least one coloring material, characterized by providing the additive reagents, comprising bacitracin (antibiotic substance), potassium tellurite and at least one coloring material in dried form on (a) support(s); and providing a sterile liquid basal medium, comprising the main nutritional ingredients dissolved in water in a separate vessel.

2. A culture medium composition according to claim 1, characterized therein that the additives comprise bacitracin, potassium-tellurite, crystal violet and trypan blue.

3. A culture medium composition according to one of the claims 1 or 2, characterized therein that the additive reagent-containing support(s) comprise(s) paper, pulp, synthetic fiber, glass fiber, fabric or water absorbing plastic material of any form, optionally plate like, film like, spherical, cubic.

4. A culture medium composition according to one of the preceding claims, characterized in that (a) support(s) comprising the additive(s) are/is bonded to a non-water absorbing sheet.

## Patentansprüche

1. Nährbodenzusammensetzung zur Vermehrung von und wahlweisen Untersuchung auf das Vorhandensein von Streptococcus mutans, die folgende Bestandteile enthält: eine wäßrige Lösung von Hauptnährbestandteilen einer MSB-Nährlösung, einschließlich Tryptose(n), Pepton(en), Saccharose, Dextrose, Phosphat und Zusatzstoffen, darunter Antibiotika, Kaliumtellurit und wenigstens ein Farbstoff, dadurch gekennzeichnet, daß die Zusatzstoff-Reagenzien, darunter Bacitracin (antibiotische Substanz), Kaliumtellurit und wenigstens ein Farbstoff, in getrockneter Form auf einem Träger (auf Trägern) vorliegen, und daß ein steriles, flüssiges Grundmedium vorliegt, welches die Hauptnährbestandteile in einem separaten Gefäß in Wasser gelöst enthält.

2. Nährbodenzusammensetzung nach Anspruch 1, dadurch gekennzeichnet, daß die Zusatzstoffe Bacitracin, Kaliumtellurit, Kristallviolett und Trypan®-Blau einschließen.

3. Nährbodenzusammensetzung nach einem der Ansprüche 1 oder 2, dadurch gekennzeichnet, daß der (die) Träger, auf dem (denen) sich die Zusatzstoff-Reagenzien befinden, Papier, Zellstoff, synthetische Fasern, Glasfasern, Stoff- oder wasserabweisendes Plastikmaterial jeglicher Form, wahlweise als Platte, Film, Kugel, Würfel, einschließt (einschließen).

4. Nährbodenzusammensetzung nach einem der vorangehenden Ansprüche, dadurch gekennzeichnet, daß der (die) Träger, auf dem (denen) sich der (die) Zusatzstoff(e) befindet (befinden), auf ein nicht-wasserabsorbierendes Blatt geklebt ist (sind).

## Revendications

1. Composition de milieu de culture pour propager et éventuellement tester la présence de Streptococcus mutans, comprenant une solution aqueuse d'ingrédients nutritionnels essentiels d'un bouillon de culture MSB incluant tryptose(s), peptone(s), sucrose, dextrose, phosphate et additifs, comprenant des antibiotiques, de la tellurite de potassium et au moins une matière colorante, caractérisée par le fait qu'il est prévu des réactifs additifs, comprenant bacitracine (substance antibiotique), tellurite de potassium et au moins une matière colorante à l'état sec, sur un (des) support(s), et qu'il est prévu un milieu basal, liquide, stérile, comprenant les ingrédients nutritionnels essentiels dissous dans l'eau dans un récipient séparé.

2. Composition de milieu de culture selon la revendication 1, caractérisée par le fait que les additifs comprennent bacitracine, tellurite de potassium, violet cristal et bleu trypan.

3. Composition de milieu de culture selon l'une des revendications 1 ou 2, caractérisée par le fait que le (les) support(s) contenant le réactif additif comprend (comprennent), papier, pulpe, fibre synthétique, fibre de verre, tissu ou matière plastique de toute forme absorbant l'eau, éventuellement en forme de plaque, de film, sphérique, cubique.

4. Composition de milieu de culture selon l'une des revendications précédentes, caractérisée par le fait qu'un (des) support(s) comprenant l'additif(s) est (sont) lié(s) à une feuille n'absorbant pas l'eau.